# EUROPEAN PATENT APPLICATION

(11) **EP 4 450 072 A1**
(43) Date of publication of application: **23.10.2024**
(21) Application number: 22906263.3
(22) Date of filing: 30.11.2022
(51) Int. Cl.: A61K 31/519, A61P 9/10, A61K 9/08, A61K 9/20

(54) **USE OF AILDENAFIL OR SALT THEREOF IN PREPARATION OF MEDICINE FOR PREVENTING OR TREATING ISCHEMIC BRAIN DAMAGE**

(30) Priority: 14.12.2021 CN 202111519481
(71) Applicant: Zhenao Jinyinhua Pharmaceutical, Xianning, Hubei 437100 (CN)
(72) Inventor: LIN, Shanliang, Xianning, Hubei 437100 (CN); TIAN, Xin, Xianning, Hubei 437100 (CN); LIN, Hexi, Xianning, Hubei 437100 (CN)
(74) Representative: Michalski Hüttermann & Partner Patentanwälte mbB
(86) International application number: PCT/CN2022/135218
(87) International publication number: WO 2023/109513

(57) **Abstract**

The present application relates to the use of compound 1-[4-ethoxy-3-[5-(6,7-dihydro-1-methyl-7-oxy-3-n-propyl-1H-pyrazolo[4,3-d]pyrimidine)] benzenesulfonyl]-3,5-dimethylpiperazine, particularly in the preparation of drugs for the prevention and treatment of diseases caused by cerebral ischemia, particularly ischemic cerebral neuron damages and necrosis.

## Description

### TECHNICAL FIELD

The present application relates to the pharmaceutical field, specifically to the use of compound 1-[4-ethoxy-3-[5-(6,7-dihydro-1-methyl-7-oxy-3-n-propyl-1H-pyrazolo[4,3-d] pyrimidine)]benzenesulfonyl]-3,5-dimethylpiperazine, particularly in the preparation of drugs for the prevention and treatment of diseases caused by cerebral ischemia, particularly for use in the treatment of ischemic brain neuron damage and necrosis.

### BACKGROUND ART

So far, there are very limited drugs officially approved clinically for the treatment of diseases caused by cerebral ischemia. t-PA is the first and only one thrombolytic drug approved by the US FDA till now for the treatment of acute cerebral ischemia. It was approved for sale in 1987, but the drug is only effective within 3 hours of onset. Beyond this time window, its effect is unclear and can lead to a higher incidence of bleeding. Edaravone, approved for marketing in Japan, protects brain cells by clearing oxygen free radicals and inhibiting lipid peroxidation. This product has significant adverse reactions and has been reported to cause severe death. The butylbenzene peptide approved for sale in China in 2004 can only be used for acute cerebral ischemia. The above-mentioned drugs are only used for acute cerebral ischemia and have limited therapeutic effects.

The compound involved in the present application is disclosed in patent CN1127506C and its use in the treatment of erectile dysfunction drugs is also disclosed. There have been no reports on the prevention and treatment of cerebral ischemia related diseases till now.

At present, the marketed product of Aildenafil Citrate Tablets is tablet. However, oral administration of tablets has the disadvantages of long time for disintegration and dissolution process, long time for blood drug concentration to reach peak, slow onset of effect, and difficulty in administration for patients with serious illnesses (such as stroke, coma, or inability to eat). Therefore, it is necessary to develop an injection and oral solutions of Aildenafil salt to address its shortcomings in clinical use. However, the solubility of Aildenafil Citrate in water at room temperature is only about 2mg/ml. If Aildenafil Citrate is used as an injection in water, the concentration of administration is low, the total volume of administration is large, and it is difficult to be reconstituted. This limits the rate at which the blood drug concentration of Aildenafil can be increased in vivo and the administration method of Aildenafil injection, for example, limited to intravenous infusion or large volume injection.

There has not yet been developed injectable or oral solutions formulations of Aildenafil in the prior art, or developed high concentration drug compositions for injection or oral solutions. High concentration injection of Aildenafil can be administered directly and quickly, reaching a peak blood concentration in the body and taking effect quickly, avoiding large volume and long-term administration. Aildenafil injection and oral solutions have important medicinal value for a patient with acute, severe or swallowing dysfunction (such as stroke, acute thrombolysis, confusion, inability to eat, etc.).

### SUMMARY

The present application aims to provide a compound with formula (I), pharmaceutically acceptable salts thereof, or pharmaceutical compositions comprising them for use in the preparation of drugs for the treatment or prevention of human diseases caused by cerebral ischemia:

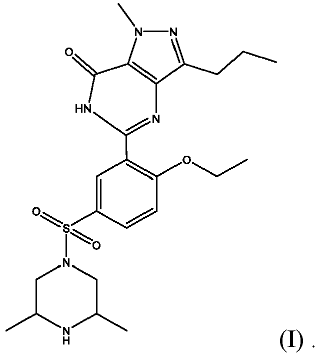

The compound of formula (I) has a cis- or trans-isomer on the 3,5-dimethyl of piperazine ring, and the cis- and trans- isomer structures are as follows:

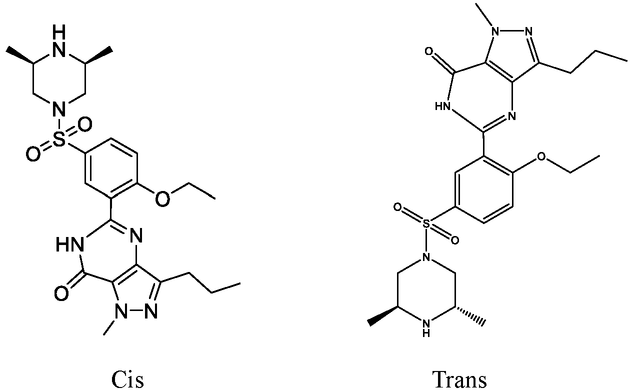

The compound of formula (I) with a cis-structure is Aildenafil, and its chemical name is 1-[3-(6,7-dihydro-1-methyl-7-oxy-3-n-propyl-1H-pyrazolo[4,3-d]pyrimidin-5-yl) -4-ethoxybenzenesulfonyl] -3,5-dimethylpiperazine.

As one of the embodiments, the present application provides the use of a pharmaceutically acceptable salt or a pharmaceutical composition comprising Aildenafil in the preparation of drugs for the treatment or prevention of human diseases caused by cerebral ischemia.

As one of the embodiments, the present application provides a pharmaceutical acceptable salt or a pharmaceutical composition thereof for the preparation of drugs for the treatment or prevention of human local ischemic cerebral infarction, human ischemic stroke, human local ischemic memory impairment and senile dementia, energy metabolism depletion of human global cerebral ischemia, or cerebral ischemic neurological symptoms caused by human brain trauma.

As one of the embodiments, the pharmaceutically acceptable Aildenafil salts of the present application include citrate, sulfate, hydrochloride, phosphate, tartrate or semi tartrate, nicotinate, lactate, gluconate, maleate or aspartate; Preferably citrate, sulfate, hydrochloride, phosphate, tartrate or semi-tartrate.

As one of the embodiments, the Aildenafil tartrate or semi-tartrate described in the present application is D-tartrate or semi D-tartrate, L-tartrate or semi L-tartrate, DL-tartrate or semi DL-tartrate, meso tartrate or semi meso tartrate; preferably Aildenafil D-tartrate or Semi D-tartrate, or Aildenafil L-tartrate or Semi L-tartrate.

As an exemplary example in the present application, the semi L-tartrate (2:1) of 1-[4-ethoxy-3-[5-(6,7-dihydro-l-methyl-7-oxy-3-n-propyl-1H-pyrazolo [4,3-d] pyrimidine)] benzenesulfonyl] -cis-3,5-dimethylpiperazine (Aildenafil) is as follows:

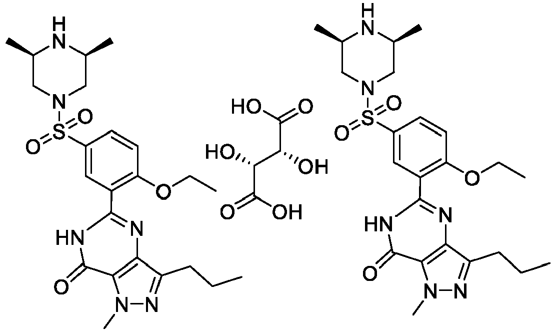

The L-tartrate (1:1) of 1-[4-ethoxy-3-[5-(6,7-dihydro-1-methyl-7-oxy-3-n-propyl-1H-pyrazolo[4,3-d]pyrimidine)]benzenesulfonyl]-cis-3,5-dimethylpiperazine (Aildenafil) has the following structure:

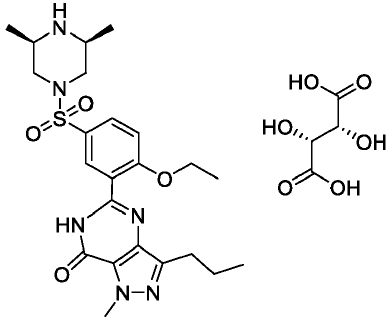

The pharmaceutical composition comprising either Aildenafil or pharmaceutically acceptable salts thereof in the present application can be various drug formulations in the art, such as but not limited to oral formulations, suppositorys, topical formulations, injections, or compound formulations. The drugs may include Aildenafil or pharmaceutically acceptable salts thereof, or pharmaceutical compositions comprising any one of them and pharmaceutically acceptable carriers. The preferred effective dosage range for human is 0.1~20mg/kg/day, administered once or multiple times per day. Preferably, the oral dosage for human is 0.2mg/kg/day~10mg/kg/day, and the injection dosage for human is 0.1mg/kg/day~5mg/kg/day. As an exemplary example, the dosage of oral administration can be 0.25mg/kg/day, 1.0mg/kg/day, or 2.0mg/kg/day. the dosage of injection administration can be 0.2mg/kg/day, 0.5mg/kg/day, or 1.0mg/kg/day.

The present application, as one of the embodiments, the oral preparation includes tablets, granules, capsules or oral solutions, and an injection includes injection solution and freeze-dried powder. As one of the embodiments, the pharmaceutical composition containing Aildenafil or its pharmaceutically acceptable salt according to the present application is oral or injectable preparation; preferred tablets, oral solutions, or injection solutions.

In the use of the present application, as one of the embodiments, the pharmaceutically acceptable salt of Aildenafil compound is Aildenafil citrate.

In the use of the present application, as one of the embodiments, when the drug combination is in tablet form, it may include Aildenafil citrate, microcrystalline cellulose, anhydrous calcium hydrogen phosphate, cross-linked carboxymethyl cellulose sodium, and magnesium stearate, preferably including Aildenafil citrate 420g, appropriate amount of microcrystalline cellulose, appropriate amount of anhydrous calcium hydrogen phosphate, appropriate amount of cross-linked carboxymethyl cellulose sodium, and appropriate amount of magnesium stearate.

The present application also provides a pharmaceutically acceptable salt of Aildenafil, wherein the pharmaceutically acceptable salt of Aildenafil is Aildenafil phosphate or Aildenafil tartrate.

As one of the embodiments, the Aildenafil tartrate described in the present application is D-tartrate, L-tartrate, DL-tartrate, or meso tartrate, preferably D-tartrate or L-tartrate.

In order to solve the problem of solubility of Aildenafil, the inventor accidentally discovered in experiments that Aildenafil can be made into phosphate or tartrate salts, which can greatly increase solubility: the solubility of Aildenafil phosphate in room temperature water reaches nearly 50mg/ml, and the pH value of the aqueous solution is between 5-6, close to neutral; and the solubility of Aildenafil L-tartrate in room temperature water is about 50mg/ml, while the solubility of Aildenafil gluconate in room temperature water is actually less than 0.2mg/ml. That is, the solubility of Aildenafil L-tartrate and phosphonate in room temperature water is more than 200 times higher than that of Aildenafil gluconate. Not only that, the dissolution of Aildenafil phosphate and L-tartrate in water is very rapid, and the aqueous solution is very stable. After 30 minutes of high-temperature and high-pressure sterilization at 121°C, no impurities are produced. Therefore, the issue of high solubility of high concentration injection and oral solutions of Aildenafil was resolved unexpectedly.

The present application provides, on the one hand, high solubility and high stability of Aildenafil salts in water-Aildenafil phosphate and tartrate. Aildenafil and tartaric acid can form salts in a molar ratio of 1:1 or 2:1. Tartaric acid can be D-tartaric acid, L-tartaric acid, meso tartaric acid, or DL-tartaric acid.

On the other hand, the present application also provides the use of Aildenafil phosphate and tartrate in the preparation of Aildenafil injections and solutions. The solubility of Aildenafil phosphate is close to 50mg/ml, and the pH value of the aqueous solution is close to neutral. The solution has good stability and is very suitable for injection use. The solubility of Aildenafil tartrate in water at room temperature is greater than 50mg/ml, and is very suitable for the preparation of Aildenafil high concentration injections and solutions. After the preparation of freeze-dried powder injection with Aildenafil phosphate and tartrate, the high concentration Reconstitution time is very short, and the solution is clear, which cannot be achieved by other salts or other solubilization methods. It is the best choice for preparing Aildenafil freeze-dried powder injection.

Compared with the prior art, the phosphate and tartrate of Ardenafil provided by the present application has the following beneficial effects:
1. The Aildenafil phosphate and tartrate of the present application are easily soluble in water at room temperature, dissolve quickly, and their solubility surprisingly increases by more than 200 times compared to other salts such as gluconate, therefore this can achieve rapid intravenous administration of Aildenafil injection in small volumes, greatly improving the rate of blood drug concentration increase and shortening the time for drug onset. They are particularly suitable for patients who require rapid administration (such as stroke, acute embolism, etc.) and those with swallowing disorders.
2. The Aildenafil phosphate and tartrate of the present application have high solubility in water, providing the possibility for the preparation of high concentration injections. It can achieve rapid intravenous administration in small volumes, avoid long-term or multiple injections in large volumes, and reduce side effects caused by large volume or multiple injections, such as burden on the body caused by high volume, toxicity caused by infusion of osmotic agents such as excessive sodium chloride and glucose.
3. The Aildenafil phosphate and tartrate of the present application has good stability, low degradation impurities, and fast reconstitution speed when used to make freeze-dried powder needles, and there is no drug precipitation at high concentrations.
4. The synthesis process for Aildenafil phosphate and tartrate of the present application is simple, the yield of its salt is nearly 100%. The process is stable, has good reproducibility, is easy to operate, has strong controllability, and is suitable for industrial production.

The present application also provides a method for preparing Aildenafil or a pharmaceutically acceptable salt thereof, in which the method includes cyclization of 4-[2-ethoxy-5-(cis-3,5-dimethylpiperazine-1-sulfonyl) benzamide] -1-methyl-3-n-propylpyrazol e-5-formamide to synthesize Aildenafil, where the base catalyst is sodium tert-butoxide.

### BRIEF DESCRIPTION OF THE DRAWINGS

FIG. 1: Aildenafil L-tartrate-HNMR in Example 3.
FIG. 2: Blank water in Example 18.

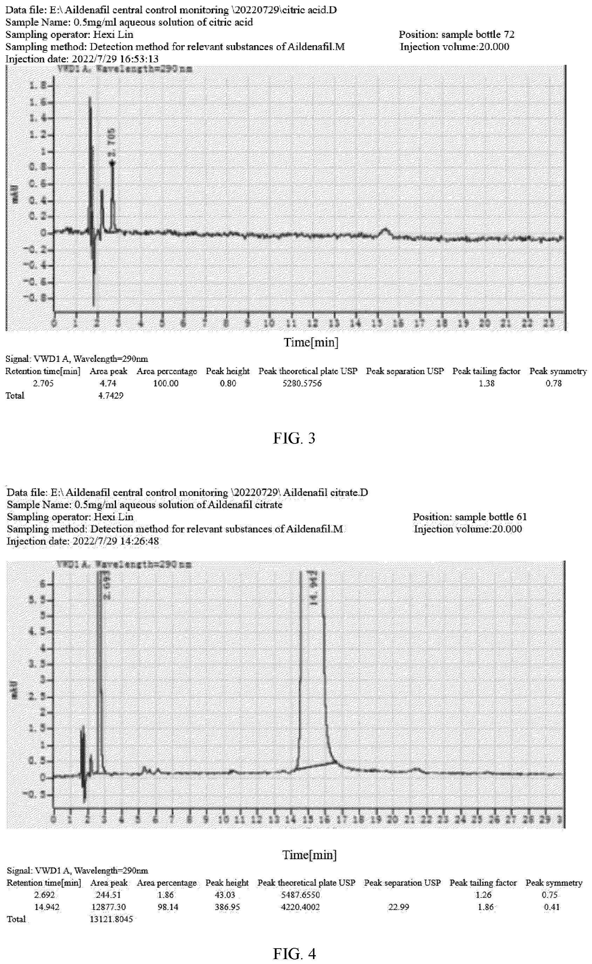

FIG. 3: Citric acid in Example 18.
FIG. 4: Aildenafil citrate in Example 18.

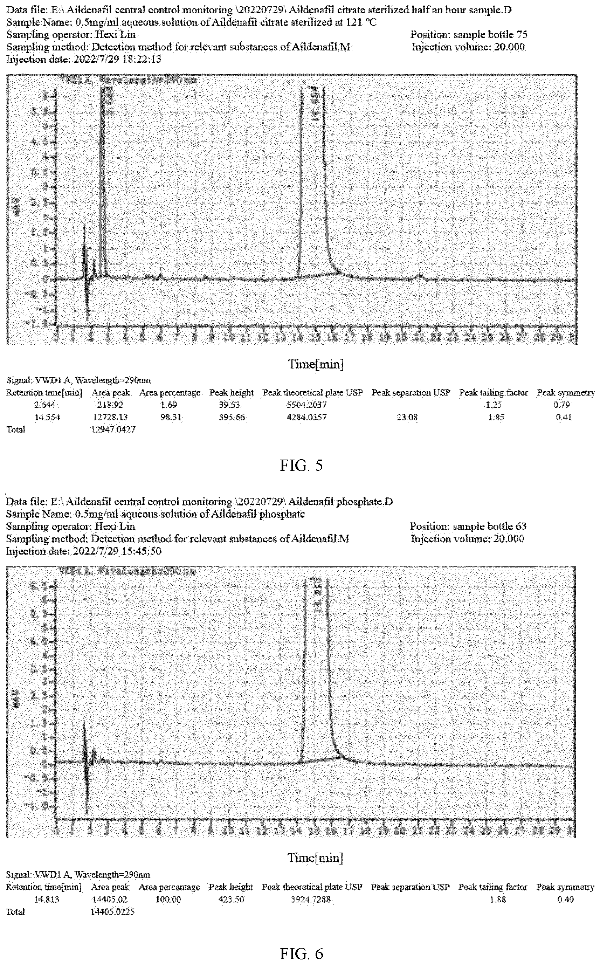

FIG. 5: Aildenafil citrate sample after sterilization for half an hour in Example 18.
FIG. 6: Aildenafil phosphate in Example 18.

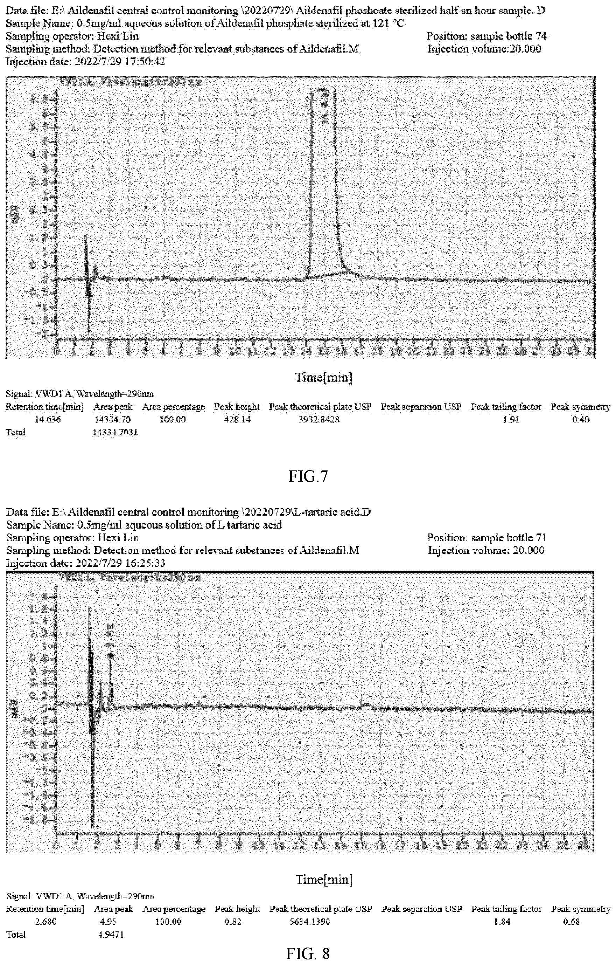

FIG. 7: Aildenafil phosphate sample after sterilization for half an hour in Example 18.
FIG. 8: L-tartaric acid in Example 18.

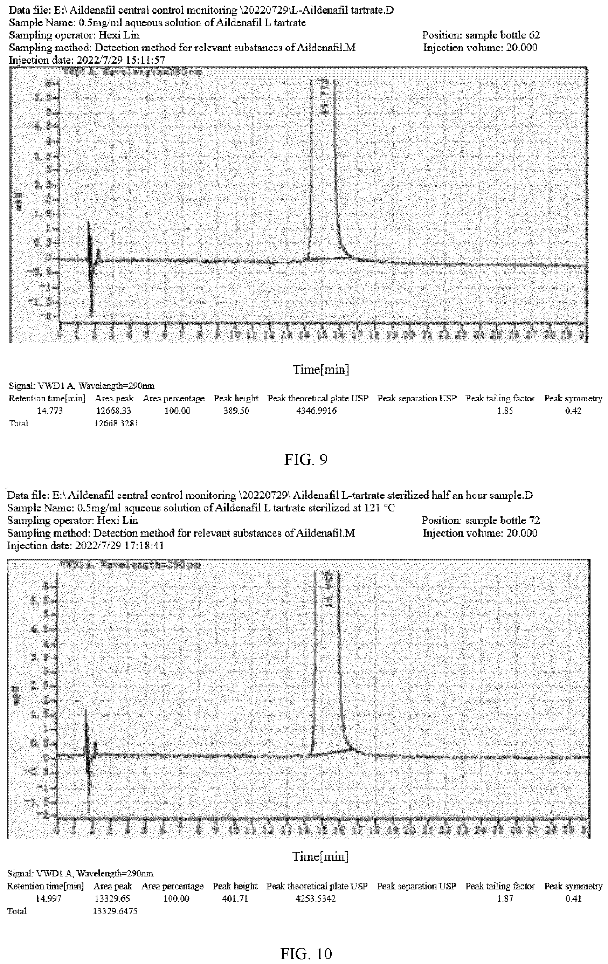

FIG. 9: Aildenafil L-tartrate in Example 18.
FIG. 10: Aildenafil L-tartrate sample after sterilization for half an hour in Example 18.
FIG. 11: Weight monitoring results of rats in Example 21.
FIG. 12: Typical legend of ADLS1026 treatment of cerebral infarction range for acute ischemic stroke in rats in Example 24, including A: model control group, B: low-dose group, C: medium dose group, and D: high-dose group.

### DETAILED DESCRIPTION

The present application will be further explained by examples below. It should be understood that the examples of the present application are not intended to limit the present application, and any simple improvements made to the present application under the premise of the present application are within the scope of protection claimed by the present application.

### Example 1 Preparation of Aildenafil and Aildenafil Citrate

### Step 1. Preparation of 5-chlorosulfonyl-2-ethoxybenzoic acid

23 ml of sulfoxide chloride was added to a 250 ml four-necked bottle under nitrogen protection, with a freezer bath temperature of -5 °C, and 81 ml of chlorosulfonyl chloride was added dropwise. 2-ethoxybenzoic acid was dissolved in a hot water bath to form a solution, and 52.5g of 2-ethoxybenzoic acid was weighted and added dropwise, releasing heat. After adding 2-ethoxybenzoic acid, stirring and the reaction is overnight. The reaction solution was brown and viscous. The reaction solution was removed and placed at room temperature.

A 1000 ml three-necked bottle was placed in the frozen solution, added with 750 ml of water, and frozen to 3 °C. The reaction solution was added dropwise at a temperature of less than 10 °C. After adding dropwise the reaction solution, a white solid was precipitated. At beginning, the solid was a bit sticky to the wall, later it could be stirred and dispersed. After adding, stirring was performed at room temperature for at least 1 hour. The solution was filtered, washed with 50 ml * 3 water at room temperature, and dried by suction to obtain a white crystalline solid with a wet weight of 68.8g. The white crystalline was dried overnight at room temperature. The melting point was measured to be 113-116 °C.

### Step 2: Preparation of 2-ethoxy-5-(cis-3,5-dimethylpiperazine-1-sulfonyl) benzoic acid (III).

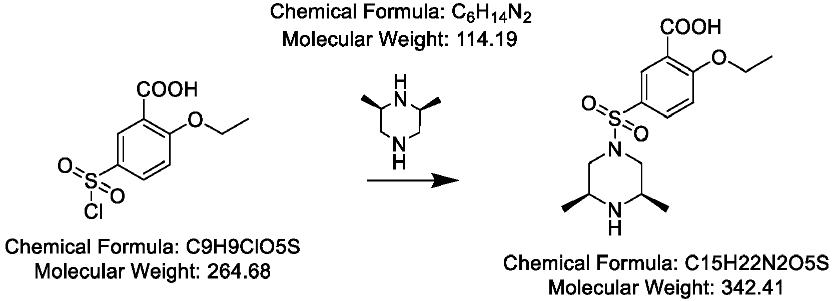

170 ml of water was added to a 1000 ml three-necked bottle, cooled down to 5 °C, added with the product 5-chlorosulfonyl-2-ethoxybenzoic acid with a wet weight of 60g obtained in the step 1, and dispersed. Cis-2,6-dimethylpiperazine was added under stirring in batches, which released heat from the reaction. The reaction temperature was controlled to be less than 10 °C. After adding, the freezing was stopped, and the solution was stirred overnight, having a measured pH 10. The solution was stirred for crystallization for 2 hours, filtered, washed with ice water three times, and dried by suction to obtain 95.0g of wet white product. The white product was dried with air at 50 °C, sampled, and tested. The pH of the filtrate was adjusted to 6-7 with 85% phosphoric acid to precipitate crystals. After adjustment, the solution was stirred for 1 hour, filtered, and washed with water to obtain white crystals with a wet weight of about 30g. The white crystal was dried with air at 50 °C to obtain a total of 67.0g. Grinded, added with 200 ml of acetone, refluxed for 1 hour, almost insoluble, cooled to room temperature, left to stand for 1 hour, filtered, rinsed with 50 ml * 3 of acetone, dried by suction and air dried at 50 °C for 2 hours, resulting in 67.2g of almost white crystals. The melting point was 258.1~258.7 °C.

### Step 3: Preparation of 2-ethoxy-5-(cis-3,5-dimethylpiperazine-1-sulfonyl) benzoyl chloride

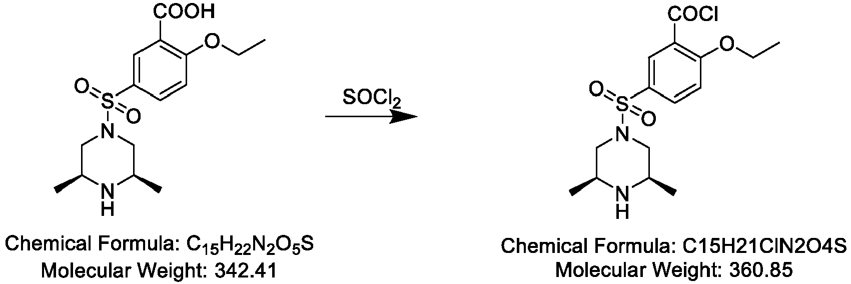

67.2g of the product obtained in the step 2 was added to a 500 ml single-necked bottle, added with 150 ml of sulfoxide chloride, dissolved and bubbled, heated up to 50 °C, dissolved to be clear solution, heated and refluxed for 3 hours. The released hydrogen chloride gas is absorbed with alkaline water, spin dried, added with 300 ml of ethyl acetate, shaken to form a uniform solution, which quickly precipitate yellow crystals. The yellow crystal was left to stand for crystallization overnight, filtered, rinsed with ethyl acetate, dried by suction and air dried at room temperature for 1 hour, and then air dried at 50 °C for 2 hours to obtain 65.0g of yellow crystal product.

### Step 4 Preparation of 4-[2-Ethoxy-5-(cis-3,5-dimethylpiperazine-1-sulfonyl) benzamide] -1-methyl-3- n-propylpyrazole-5-formamide

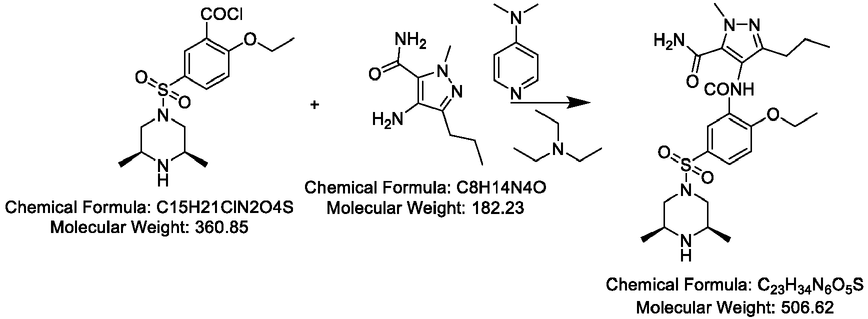

1.0g of DMPA, 30.0g of pyrazole formamide, and 320 ml of dichloromethane were added to a 1000 ml three-necked bottle, dissolved to be clear, added with 34.0g of triethylamine, and cooled under stirring to 0 °C. The temperature of the reaction solution was -3 °C. A total of 63.5g of the product obtained in step 3 was added in batches, which released heat. The temperature of the reaction solution was controlled to be less than 5°C. After adding, the freezing was stopped, the temperature was raised to room temperature, and stirring reaction more than 2 hours. The solution was distilled under reduced pressure until drying, added with 300 ml of water, stirred to crystallize, and filtered to obtain a reddish brown filtrate and a yellow product. The product was washed with water by 100 ml * 3 times and with ethyl acetate by 100 ml * 3, washed off the red with ethyl acetate and obtain basically colorless filtrate, and dried by suction to obtain 88.6g wet weight of loose off-white crystals. The crystal was firstly air dried at room temperature, and then air-blowing dried at 50°C for 3 hours to obtain 69.0g, with a measured melting point of 194.3-195.1°C.

69g of the crude product obtained above was added to a 1000 ml single-necked bottle, added with 560 ml of water, and 280 ml of methanol, heated and refluxed. It was found that it could not be dissolved completely. 100 ml of methanol was added, refluxed for dissolution to clear, filtered while hot, and white crystals were quickly precipitated from the filtrate. The filtrate was left to stand for 5 hours, filtered, washed with 150 ml methanol: water at a ratio of 1:2, dried by suction, and dried with air at 50 °C until constant weight, resulting in 60.0g. Measure the melting point from 199.4 to 199.9 °C.

Detection method: using octadecylsilane bonded silica gel as a filler; 0.05mol/L triethylamine phosphate (diluting 7 ml of triethylamine to 1000 ml with water, and adjust pH to 3.0 with phosphoric acid)-methanol acetonitrile (58:25:17) as the mobile phase; and a detection wavelength of 290nm.

### Step 5: Preparation of cyclization of 5-[2-ethoxy-5-(cis-3,5-dimethylpiperazine-1-sulfonyl) phenyl] -1-methyl-3-n-propyl-7,6-dihydro-1H-pyrazolo [4,3-d] pyrimidin-7-ketone.

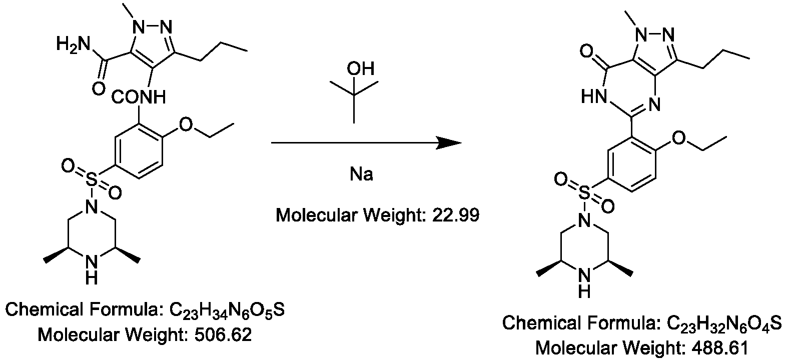

300 ml of tert-butanol was added to a 1000 ml dry single-necked bottle, and added with 3.0g of metal sodium in batches under room temperature while magnetic stirring. After adding the metal sodium, the bottle was heated to 70°C and stirred with magnetic stirring until the metal sodium disappears (sodium tert-butoxide is generated in situ, or commercial sodium tert-butoxide products can be directly added). 57g of the product obtained in the step 4 was added in batches while stirring. After adding, stirring and refluxing were performed for at least 8 hours. The reaction solution was cooled to room temperature, added dropwise with 500 ml of water at room temperature, and stirred evenly. Vacuum distillation was performed to remove tert-butanol, and the solution was stirred at room temperature for more than 2 hours, filtered, washed with water until neutral, and dried with air at 50°C to obtain 40g of crude product. The crude product was added with 1000 ml of ethanol, refluxed to obtain clear solution, and cooled to crystallize. The solution was filtered and the crystal was air-blowing dried at 50°C to obtain 35.1g of Aildenafil product.

### Step 6 Preparation of Aildenafil Citrate

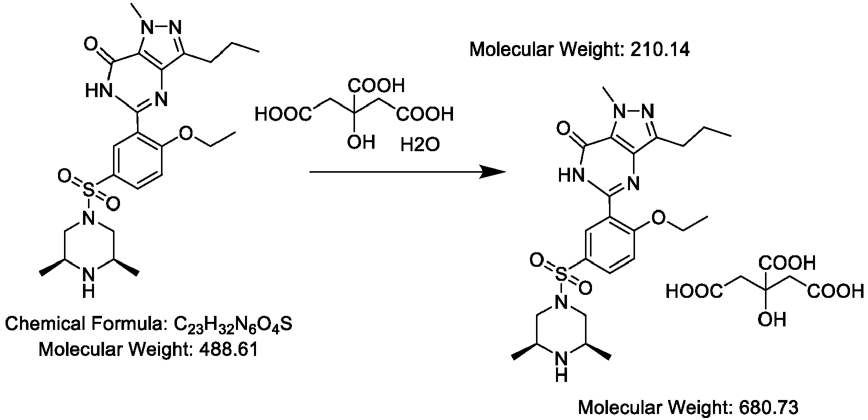

870 ml of anhydrous ethanol, 34.8g of Aildenafil, and 17.4g of citric acid monohydrate were added to a 1000 ml single-necked bottle, heated and refluxed for 1.5 hours. Heating was stopped. The solution was stirred to cool naturally to room temperature for crystallization for more than 5 hours. The solution was filtered, and the crystal was rinsed with ethanol, dried by suction, and air dried at 50°C to constant weight. The product of Aildenafil citrate was 47.4g, which was a white crystal with a yield of 97.7%, a melting point of 210.2~211.9 °C (melting decomposition), and a purity of >99.9%. It was crushed through an 80 mesh sieve to obtain a white powdered product. The mass spectrometry [M+H]+was 489.50.

### Example 2: Preparation of Aildenafil Citrate

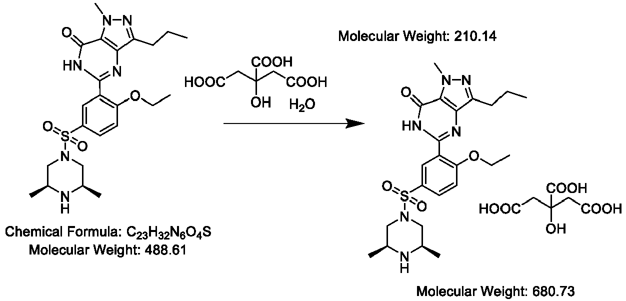

60 ml of anhydrous ethanol and 2.45g of Aildenafil were added to a 250 ml single-necked bottle, heated and refluxed to dissolve and be clear, and added a solution containing 1.1g of citric acid monohydrate and 5 ml of anhydrous ethanol. Heating was stopped, and the solution was stirred to naturally cool to room temperature for crystallization for more than 5 hours. The solution was filtered to obtain a crystal, and the crystal was rinsed with ethanol, dried by suction, and dried with air-blowing at 60 °C until constant weight to obtain 3.33g of the product, which was a white crystal with a yield of 97.7%, a melting point of 216.5-217.0°C (melting decomposition), and a purity of 100%.

### Example 3 Preparation of Aildenafil L-Tartrate

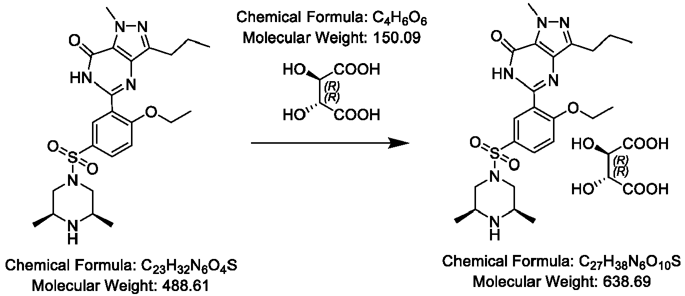

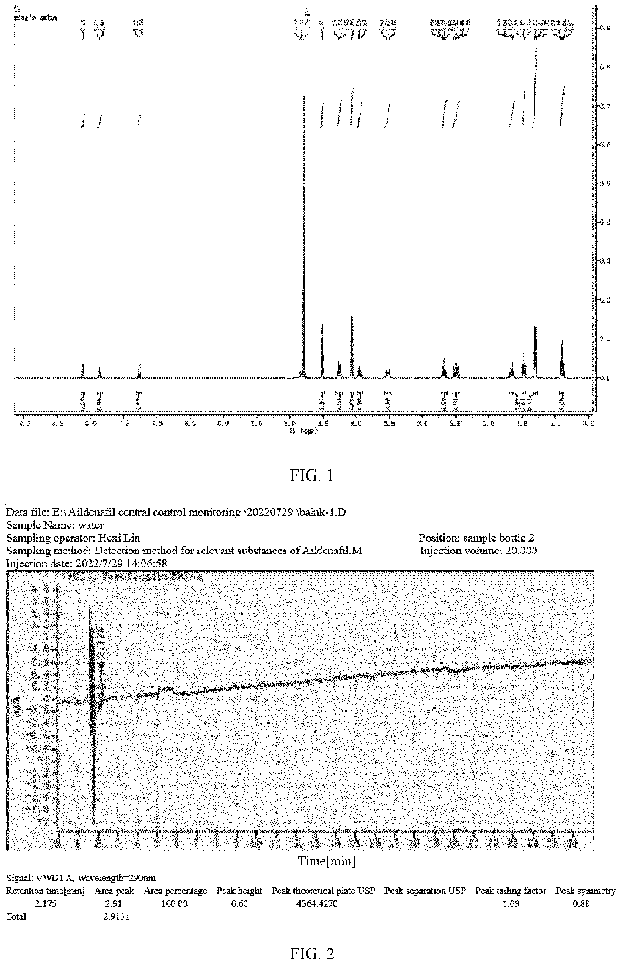

2.45g of Aildenafil white crystalline powder was added to a 250 ml single-necked bottle, added with 60 ml of anhydrous ethanol and heated to reflux for dissolution to clear. The solution was added with a clear solution of 0.77g of L-tartaric acid and 5 ml of anhydrous ethanol to precipitate a solid. After this, reflux was performed for 30 minutes. The solution was naturally cooled for crystallization, and stirred overnight at room temperature. The solution was suction filtered, and the crystal was rinsed twice with 5 ml anhydrous ethanol, then dried by suction to obtain a white solid. The crystal was air dried at 60 °C to constant weight. The crystal was baked to constant weight to obtain 3.2g of white crystals, with a yield of 100% and a melting point of 205.8-207.4C. The HNMR image is shown in FIG. 1.

### Example 4: Preparation of Aildenafil Phosphate

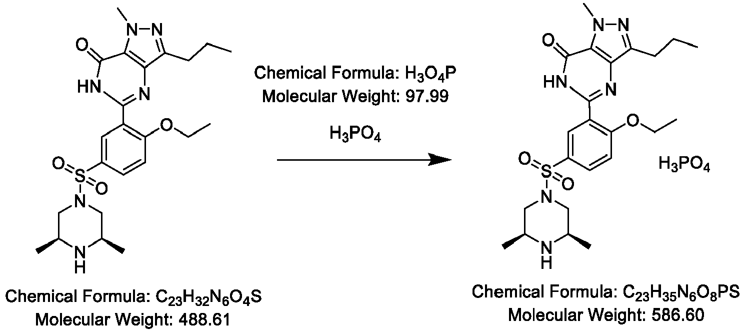

4.89g of Aildenafil white crystalline powder was added to a 250 ml single-necked bottle, added with 120 ml of anhydrous ethanol, heated and refluxed to dissolve and be clear, added with 1.15g of 85% phosphoric acid to obtain a turbid reaction solution, with solid precipitated. After adding, reflux was performed for 30 minutes. Natural cooling was performed to crystallize, and the solution was stirred overnight at room temperature. The solution was suction filtered and the crystal rinsed twice with 5 ml of anhydrous ethanol, then dried by suction to obtain a white solid. The white solid was dried with 80 °C air to constant weight. It was baked to constant weight to obtain 5.8g of white crystals, with a yield of 98.8% and a melting point of 225.3-227.0°C.

### Example 5: Preparation of Aildenafil Maleate

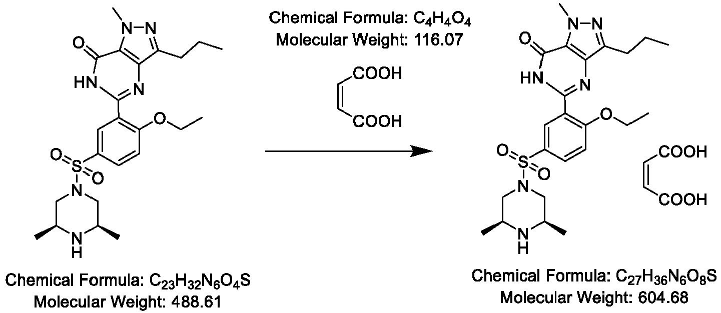

2.45g of Aildenafil white crystalline powder was added to a 250 ml single-necked bottle, added with 60 ml of anhydrous ethanol, heated and refluxed to dissolve, and added with 0.59g of maleic acid, with solid precipitated. After adding, reflux was performed for 30 minutes. Natural cooling was performed to crystallize, and the solution was stirred overnight at room temperature. The solution was suction filtered and the crystal was rinsed twice with 5 ml of anhydrous ethanol, then dried by suction to obtain a white solid. The white solid was dried with air-blowing at 60°C to constant weight. It was dried to constant weight to obtain 2.8g of white crystals, with a yield of 92.4% and a melting point of 221.0-222.3 °C.

### Example 6 Preparation of Aildenafil Gluconate

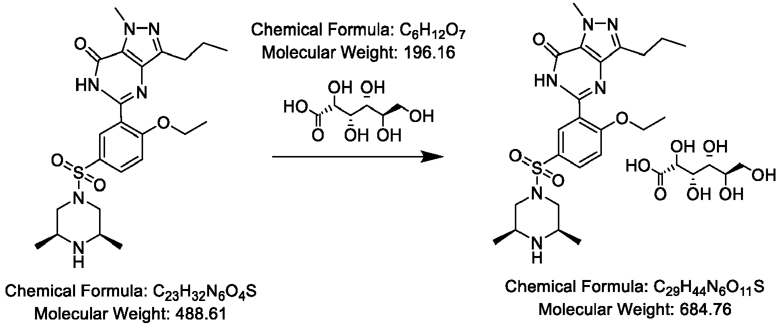

1.00g of Aildenafil white crystalline powder was added to a 250 ml single-necked bottle, added with 26 ml of anhydrous ethanol, heated and refluxed to dissolve and be clear, and added with 0.81g of gluconic acid solution (49-53%) dropwise. Reflux was performed for 30 minutes after adding. Natural cooling was performed to crystalize to obtain turbid solution due to precipitation of crystals at about 45°C. The solution was stirred overnight at room temperature. It was suction filtered and washed twice with 5 ml of anhydrous ethanol, then dried by suction to obtain a white solid. It was dried via air-blowing at 60 °C to constant weight. It was baked to constant weight to obtain 0.6g of white crystals, with a yield of 42.8% and a melting point of 195.0-195.8 °C.

### Example 7 Preparation of Aildenafil Lactate

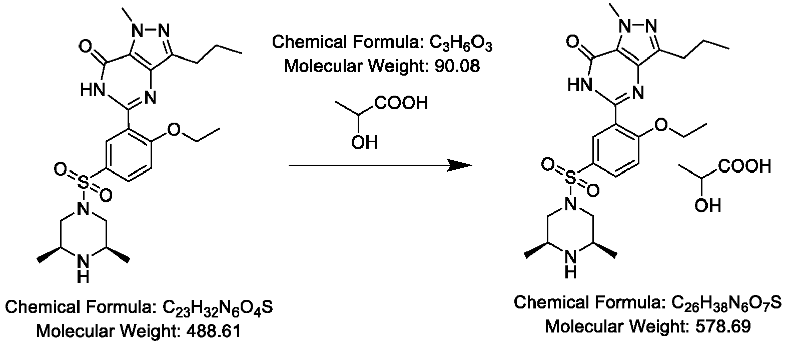

2.45g of Aildenafil white crystalline powder was added to a 250 ml single-necked bottle, added with 60 ml of anhydrous ethanol, heated and refluxed to dissolve and be clear, and added with 0.51g of lactic acid. After adding, dissolve quickly and reflux was performed for 30 minutes. Natural cooling was performed to crystalize, which started at 40 °C, and the solution was stirred overnight at room temperature, suction filtered and washed twice with 5 ml of anhydrous ethanol, then dried by suction to obtain a white solid. It was air-blowing dried at 60 °C to constant weight. It was baked to constant weight to obtain 2.2g of white crystals, with a yield of 75.9% and a melting point of 180.6-181.8 °C.

### Example 8: Preparation of Aildenafil Aspartate

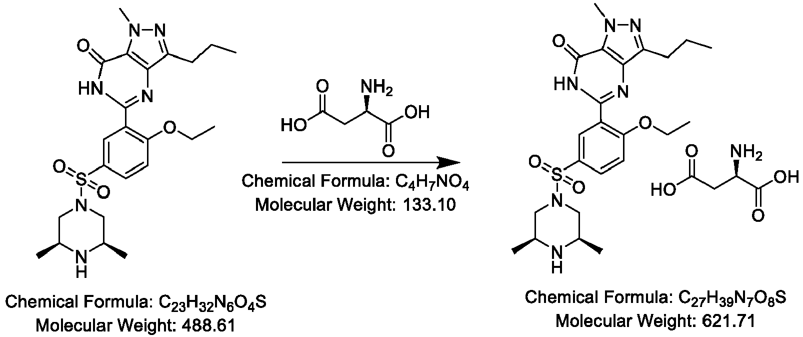

1.00g of Aildenafil white crystalline powder was added to a 250 ml single-necked bottle, added with 26 ml of anhydrous ethanol, heated and refluxed to dissolve and be clear, added with 0.31g of aspartic acid. After adding, dissolve quickly to clear and reflux was performed for 30 minutes. Natural cooling was performed to crystalize, and the solution was stirred overnight at room temperature, suction filtered and washed twice with 5 ml of anhydrous ethanol, then dried by suction to obtain a white solid. It was air-blowing dried at 60°C to constant weight. It was baked to constant weight to obtain 1.0g of white crystals, with a yield of 78.9% and a melting point of 193.1-194.4°C.

### Example 9 Preparation of Aildenafil Nicotinate

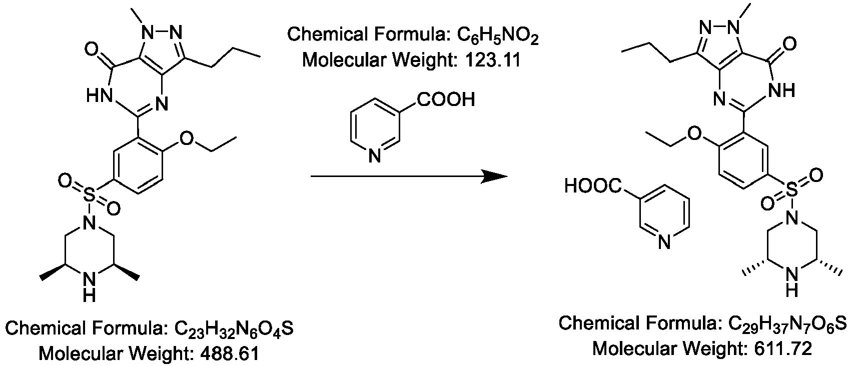

2.45g of Aildenafil white crystalline powder was added to a 250 ml single-necked bottle, added with 60 ml of anhydrous ethanol, heated and refluxed to dissolve and be clear, added with 0.62g of niacin. After adding, dissolve quickly to clear and reflux was performed for 30 minutes. Natural cooling was performed to crystalize, which started at around 45°C, and stirring at room temperature overnight, suction filtered and wash twice with 5 ml of anhydrous ethanol, then dried by suction to obtain a white solid. It was air dried at 60 °C to constant weight. It was baked to constant weight to obtain 2.8g of white crystals, with a yield of 91.5% and a melting point of 204.0 °C-205.0 °C.

### Example 10: Preparation of Aildenafil Hydrochloride

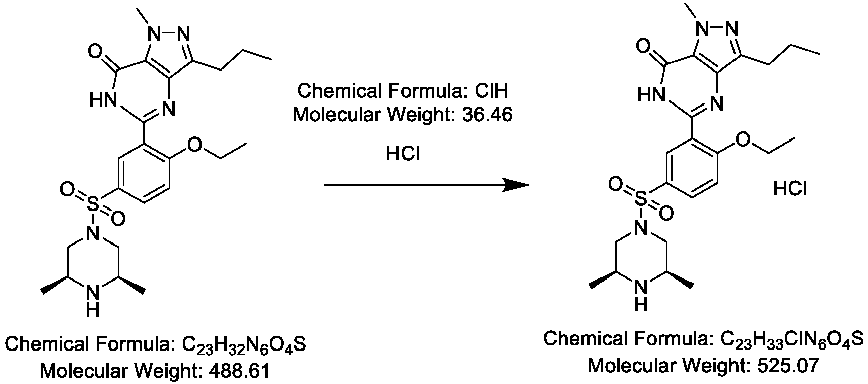

4.89g of Aildenafil white crystalline powder was added to a 250 ml single-necked bottle, added with 120 ml of anhydrous ethanol, heated and refluxed to dissolve and be clear, added with 1.01g of 36.5% hydrochloric acid to precipitate solid, reflux was performed for 30 minutes after adding. Natural cooling was performed to crystalize, and the solution was stirred overnight at room temperature, suction filtered and washed twice with 5 ml of anhydrous ethanol, then dried by suction to obtain a white solid. It was air-blowing dried at 60 °C to constant weight. It was baked to constant weight to obtain 5.2g of white crystals, with a yield of 99.0% and a melting point of>250 °C.

### Example 11 Preparation of Aildenafil Sulfate

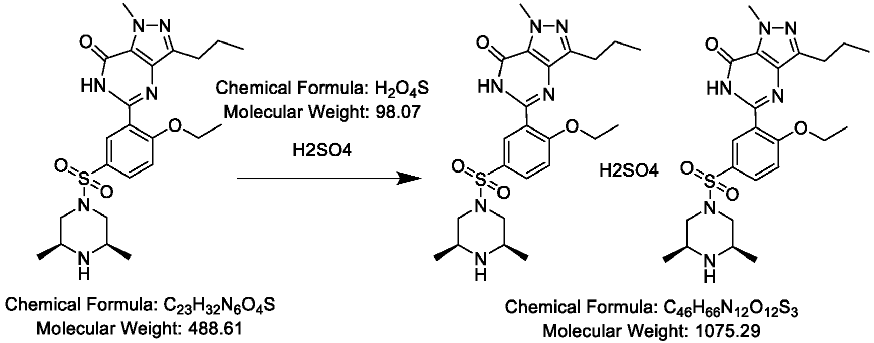

4.89g of Aildenafil white crystalline powder was added to a 250 ml single-necked bottle, added with 120 ml of anhydrous ethanol, heated and refluxed to dissolve and be clear, added with 0.52g of concentrated sulfuric acid, precipitate a large amount of solid, reflux was performed for 30 minutes after adding. Natural cooling was performed to crystalize, and the solution was stirred overnight at room temperature, suction filtered and washed twice with 5 ml of anhydrous ethanol, then dried by suction to obtain a white solid. It was air dried at 60°C to constant weight. It was baked to constant weight to obtain 4.4g of white crystals, with a yield of 81.8% and a melting point of 236.4-237.8 °C.

### Example 12 Preparation of Aildenafil D-Tartrate

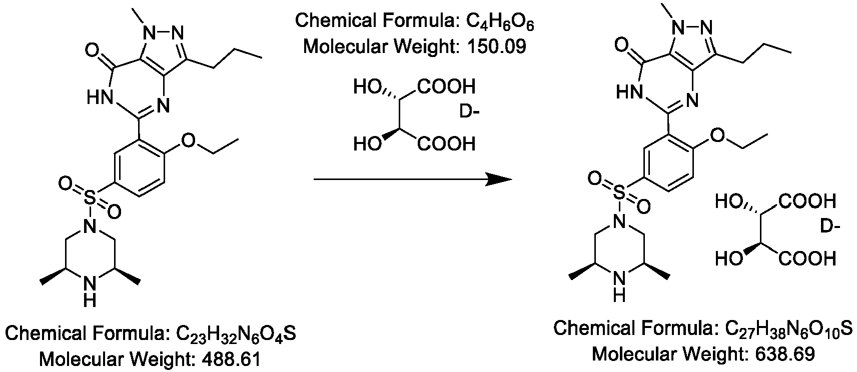

1.0g of Aildenafil white crystalline powder was added to a 250 ml single-necked bottle, added with 31 ml of anhydrous ethanol, heated and refluxed to dissolve and be clear, added a clear solution containing 0.31g of D-tartaric acid and 5 ml of anhydrous ethanol to precipitate a solid, reflux was performed for 30 minutes after adding. Natural cooling was performed to crystalize, and the solution was stirred overnight at room temperature, suction filtered and rinsed twice with 5 ml anhydrous ethanol, then dried by suction to obtain a white solid. It was air-blowing dried at 60°C to constant weight. It was baked to a constant weight to obtain 1.0g of white crystals with a melting point of 207.0-208.0°C.

### Example 13 Preparation of Aildenafil DL-Tartrate

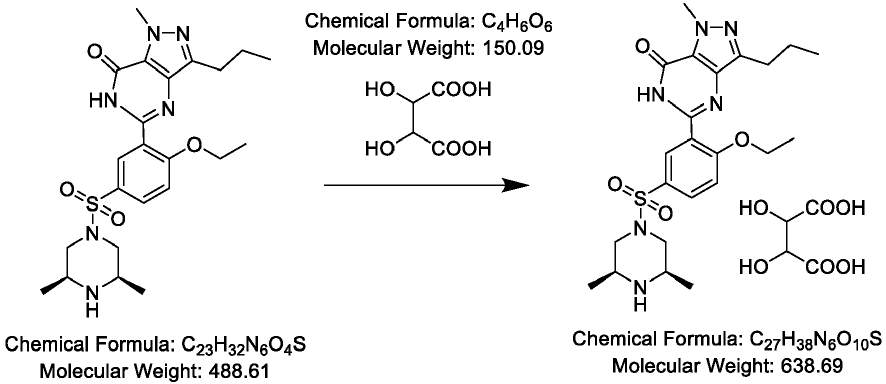

1.0g of Aildenafil white crystalline powder was added to a 250 ml single-necked bottle, added with 31 ml of anhydrous ethanol, heated and refluxed to dissolve and be clear, added a clear solution containing 0.31g of DL-tartaric acid and 5 ml of anhydrous ethanol to precipitate a solid, reflux was performed for 30 minutes after adding. Natural cooling was performed to crystalize, and the solution was stirred overnight at room temperature, suction filtered and rinsed twice with 5 ml anhydrous ethanol, then dried by suction to obtain a white solid. It was air-blowing dried at 60°C to constant weight. It was baked to a constant weight to obtain 1.0g of white crystals with a melting point of 197.0-198.0 °C.

### Example 14: Preparation of Aildenafil Meso-Tartrate

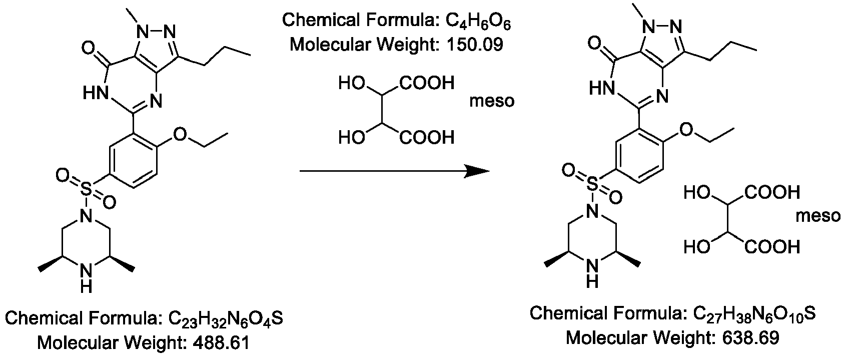

1.0g of Aildenafil white crystalline powder was added to a 250 ml single-necked bottle, added with 31 ml of anhydrous ethanol, heated and refluxed to dissolve and be clear, added a clear solution containing 0.31g of meso tartaric acid and 5 ml of anhydrous ethanol to precipitate a solid, reflux was performed for 30 minutes after adding. Natural cooling was performed to crystalize, and the solution was stirred overnight at room temperature, suction filtered and rinsed twice with 5 ml anhydrous ethanol, then dried by suction to obtain a white solid. It was air-blowing dried at 60 °C to constant weight. It was baked to constant weight to obtain 1.1g of white crystals.

### Example 15: Preparation of Aildenafil Semi L-Tartrate

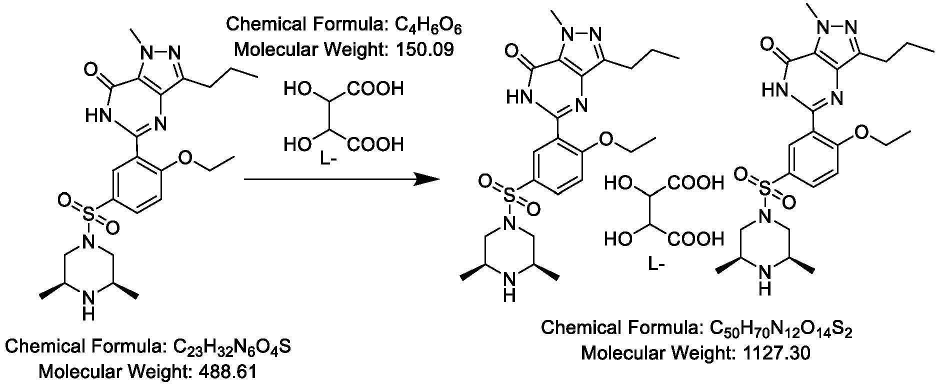

2.45g of Aildenafil white crystalline powder was added to a 250 ml single-necked bottle, added with 60 ml of anhydrous ethanol, heated and refluxed to dissolve and be clear, added a clear solution containing 0.38g of L-tartaric acid and 5 ml of anhydrous ethanol to precipitate a solid, resulting in turbid solution, and reflux was performed for 30 minutes. Natural cooling was performed to crystalize, and the solution was stirred overnight at room temperature, suction filtered and rinsed twice with 5 ml anhydrous ethanol, then dried by suction to obtain a white solid. It was air-blowing dried at 60 °C to constant weight. It was baked to constant weight to obtain 2.4g of white crystals with a yield of 85%, and a melting point of 210.0~211.5 °C.

### Example 16 Preparation of Aildenafil L-Tartrate

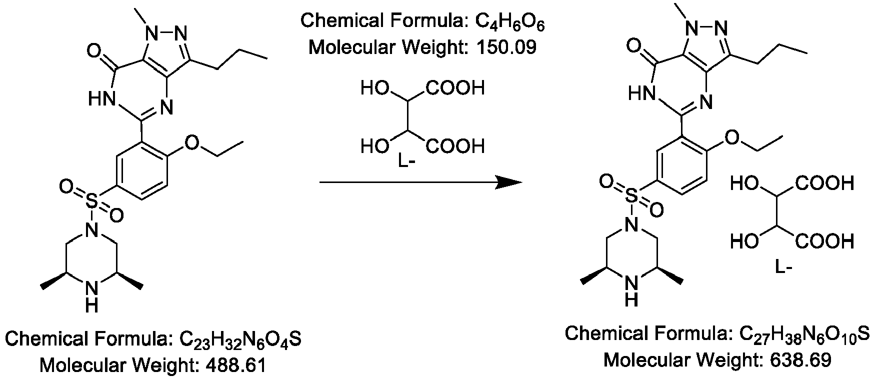

1.0g of Aildenafil white crystalline powder was added to a 250 ml single-necked bottle, added with 19 ml of 95% ethanol, heated and refluxed to dissolve and be clear, added a clear solution containing 0.32g of L-tartaric acid and 5 ml of 95% ethanol to precipitate a solid at about 5 minutes, resulting in a turbid solution, and reflux was performed for 30 minutes. Natural cooling was performed to crystalize, and the solution was stirred overnight at room temperature, suction filtered and rinsed twice with 5 ml anhydrous ethanol, then dried by suction to obtain a white solid. It was air-blowing dried at 60°C to constant weight. It was baked to constant weight to obtain 1.2g of white crystals with a yield of 91.6% and a melting point of 206.0-207.0°C.

### Example 17: Room Temperature Water Solubility of Different Aildenafil Salts

The saturation solubility of different Aildenafil salts in water was measured at room temperature, and the results are as follows:

| Saturation solubility of different Aildenafil salts in water | | |
|---|---|---|
| Aildenafil salts Name | Appearance | Saturated Solubility in water at room temperature |
| Citrate | White crystal | 2mg/ml |
| Sulfate | White crystal | 2.5mg/ml |
| Hydrochloride | White crystal | 6mg/ml |
| Phosphate | White crystal | About 50mg/ml |
| L-tartrate | White crystal | About 50mg/ml |
| Nicotinate | White crystal | 0.3mg/ml |
| Lactate | White crystal | <0.2mg/ml |
| Gluconate | White crystal | <0.2mg/ml |
| Maleate | White crystal | 0.4mg/ml |
| Aspartate | White crystal | <0.2mg/ml |
| D-tartrate | White crystal | About 50mg/ml |
| DL-tartrate | White crystal | About 50mg/ml |

Experimental results: After salifying, all of the Aildenafil salts are white crystals, but the saturation solubility of different salts in water varies greatly, and difference between the maximum and minimum solubility exceeds 200 times. Among them, L-tartrate is easily soluble in water at room temperature, with a saturation concentration of about 50mg/m after dissolution. The saturated concentration of phosphate dissolved in room temperature water was about 50mg/ml. In contrast, the maximum concentration of other salts after saturation can only reach about 6mg/ml (hydrochloride 6mg/ml). The gluconate which has a similar structure with the tartrate was almost insoluble in water at room temperature, and its concentration after saturation was less than 0.2mg/ml. The solubility of Aildenafil L-tartrate and phosphate in water was very surprising. In addition, Aildenafil L-tartrate and phosphate were dissolved very quickly in water, and can be dissolved to be clear in 15 seconds by hand shaking, which is very beneficial for the re-constitution of freeze-dried powder injection. Furthermore, in terms of the yield of salt formation, the yield of Aildenafil L-tartrate and phosphate was over 95%, which basically meets the requirements of atomic economy requirement.

### Example 18 Sterilization Stability Test for Aildenafil Citrate, Aildenafil Phosphate, and Aildenafil L-Tartrate Aqueous Solution

10mg each of Aildenafil L-tartrate, Aildenafil phosphate, and Aildenafil citrate was added to a 20 ml volumetric flask, and added with purified water to volume. They were sterilized in a 121 °C sterilizer for 30 minutes, and sampled before and after sterilization. The relevant substances were measured according to the following method:

Relevant substances: taking the samples and dissolving it in the mobile phase to prepare a solution containing 0.5mg per 1 ml, which was used as the test solution; using octadecylsilane bonded silica gel as the filler, phosphate buffer (800 ml of water, 7 ml of triethylamine added, pH adjusted to 3.0 with phosphoric acid, diluted to 1000 ml with water) -methanol acetonitrile (58:25:17) as the mobile phase, with a detection wavelength of 290nm.

Experimental results: 0.5mg/ml aqueous solution of Aildenafil L-tartrate, Aildenafil phosphate, and Aildenafil citrate was sterilized at 121 °C for 30 minutes, and no impurities were found. There was no difference in the HPLC images of the solution before and after sterilization. The HPLC images before and after sterilization are shown in FIG.s 2 to 10. The results are shown in the table below:

Comparison of Related Substances of Different Salts 0.5mg/ml Sterilized at 121°C for 30 Minutes

| Name | Relevant substances before sterilization | Relevant substances after sterilization | Conclusion |
|---|---|---|---|
| Aildenafil L-tartrate | None | None | No changes, stable sterilization |
| Aildenafil phosphate | None | None | No changes, stable sterilization |
| Aildenafil citrate | None | None | No changes, stable sterilization |

### Example 19: pH Measurement of Typical Different Aildenafil Salts Dissolved in Water

The most soluble Aildenafil salts in water were determined: Aildenafil tartrate and Aildenafil phosphate, and compared with Aildenafil citrate. The results are as follows:

| Name | Solution properties | 10mg/ml pH value | 2mg/ml pH value |
|---|---|---|---|
| D-tartrate | | 3.58 | 3.84 |
| Phosphate | Clear and colorless | 5.38 | 5.47 |
| DL-tartrate | | 3.57 | 3.90 |
| L-tartrate | | 3.56 | 3.59 |
| Citrate | | Insoluble | 4.11 |

It was unexpectedly discovered that, phosphate pH was closer to physiological pH, being suitable for injection administration.

### Example 20 Preparation of Aildenafil Citrate Formulation

Aildenafil Citrate can be made into various preparations, including tablets, granules, capsules, sublingual patches, suppositories, topical preparations, oral solutions, injections, etc. Ordinary oral rapid release tablets can be prepared as follows:
The prescription for Aildenafil Citrate tablets is as follows:
The prescription for 60mg Aildenafil tablets (calculated by alkali) is as follows (5000 tablets):

| Name | Dosage | Mass percentage (%) |
|---|---|---|
| Aildenafil Citrate | 420g | 23.33% |
| Microcrystalline cellulose | 940g | 52.2% |
| Anhydrous calcium hydrogen phosphate | 314g | 17.4% |
| Crosslinked carboxymethyl cellulose sodium | 90g | 5.00% |
| Magnesium stearate | 36g | 2.0% |
| Total | 1800 | |
| Purified water | 900g | |

The preparation process of tablets was as follows:
Microcrystalline cellulose, Aildenafil raw material, anhydrous calcium hydrogen phosphate, and cross-linked carboxymethyl cellulose sodium according to the prescribed amount (5000 tablets) were weighed, pulverized through 80 mesh sieve and mixed. The mixed pulverized and sieved materials mentioned above was added with purified water, wet granulated and mixed for 15 minutes to prepare soft materials, and then passed through 16 mesh sieve to granulation. The amount of water was adjusted according to the granulation situation. The prepared granules were dried in an oven by air-blowing at a temperature of 60°C± 5 for 4 hours and the weight loss during drying was measured. After drying weight loss qualified, the dried granules were sieved through 10 mesh sieve and sized to obtain the granules. After granulation, the mixture was poured into a 3D motion mixer and added with the prescribed amount of magnesium stearate, mixed for 100 revolutions. Samples were taken and tested. All the mixed material was loaded into a loading port of a tablet press, controlled at a loading height, and tablet pressed. The weight of the tablet was adjusted to 360mg, having a hardness of 30-60N, and a brittleness of less than 0.3%. The difference between tablets weight was controlled at (± 4%).

### Example 21: Comparative Study on the Repair Effect of Oral Administration of Aildenafil Citrate and Sildenafil Citrate on Neural Injury in MCAO Rats

### 1. Experimental drugs

Aildenafil citrate: white powder, solid, with a purity greater than 99%. Self made, batch number 20191127-1.

Sildenafil citrate (USP): white powder, solid, with a purity greater than 99%. Manufacturer AZICO BIOPHORE from India; Batch number: 4002/4/005/20.

### 2. Experimental animals

SD rats, 210-230g.

### 3. Drug configuration

Aildenafil citrate: dissolved in purified water to prepare a 2mg/ml drug solution, refrigerated at 4 °C to be used.

Sildenafil citrate: dissolved in purified water to prepare a 2mg/ml drug solution, refrigerated at 4 °C to be used.

### 4. Administration route, dosage, and time

Administration route: gavage administration;
Dosage: 4mg/kg body weight, twice per day;
Administration time: gavage administration twice in the morning and evening per day (administered immediately after modeling).

### 5. Production of MCAO models

A rat model of middle cerebral artery ischemia/reperfusion (MCAO/R) was created using the suture method.

### 6. Experimental grouping

The experimental rats were divided randomly into 4 groups, labeled as MCAO model group (referred to as model group), Sham group, treatment group 1 (Aildenafil group), and treatment group 2 (Sildenafil group), with 30 rats in each group.
① MCAO model group: cerebral ischemia-reperfusion group;
② Sham group: After surgical exposure of the carotid artery, suture was performed without ischemia-reperfusion injury;
③ Treatment group 1: MCAO model+Aildenafil citrate drug treatment group ;
④ Treatment group 2: MCAO model+Sildenafil citrate drug treatment group;

### 7. Experimental measurement indicators

### ✧ Weight monitoring

By monitoring the weight gain of rats in different groups at different times during the experimental process, the physical recovery of rats after cerebral infarction treated with different therapeutic drugs were indirectly evaluated.

### ✧ Neurological deficits (mNSS) score:

Scores were evaluated using the mNSS rat neurological deficit scoring method. The neurological deficits of the experimental rats were evaluated on 14 days after modeling.

### ✧ Behavioral experiments:

*Morris* water maze experiment: testing the learning and memory abilities of rats.

The *Morris* water maze experiment is conducted continuously for 6 days each time, with training conducted for the first 5 days and testing conducted on 6^{th} day.

For each of the tests, the rats were trained four times a day at a fixed time period for the first five days (once in the first, second, third, and fourth quadrants respectively). At the beginning of the training, a platform was placed in the first quadrant, and from any starting points of the four quadrants on the pool wall, the rats were placed into the pool, facing the pool wall. The camera system records the time when the rats found the platform and their swimming path. Four training sessions involved placing rats into water from four different starting points. If the rat finds the platform or cannot find it within 60 seconds, the experimenter will guide it to the platform, rest on the platform for 10 seconds, and then proceed to the next experiment.

The rats were removed from the platform, dried with a soft cloth, and put back into the cage. Afterwards, the next experiment on the rat was performed until the numbered animals had finished swimming, and then the next set of experiments was conducted.

This experiment was conducted on the 14^{th} day after administration. The experiment was conducted continuously for 6 days, with swimming training for the first 5 days and testing on the 6^{th} day.

### 8. Experimental results

### ✧ Weight monitoring

**Table 1**

| Day | model group (g) | Sham group (g) | Aildenafil group (g) | Sildenafil group (g) |
|---|---|---|---|---|
| D0 | 298.4 | 295.9 | 303.9 | 297.7 |
| D4 | 248.3 | 285.3 | 251.4 | 251.4 |
| D7 | 250.8 | 300.3 | 253.8 | 249.0 |
| D11 | 261.2 | 319.7 | 274.1 | 266.9 |
| D14 | 273.7 | 334.9 | 287.2 | 282.4 |
| D18 | 296.4 | 359.1 | 312.0 | 307.6 |
| D21 | 305.2 | 364.4 | 321.3 | 318.3 |
| D25 | 319.1 | 378.9 | 341.9 | 339.6 |
| D28 | 316.6 | 380.7 | 345.0 | 345.9 |

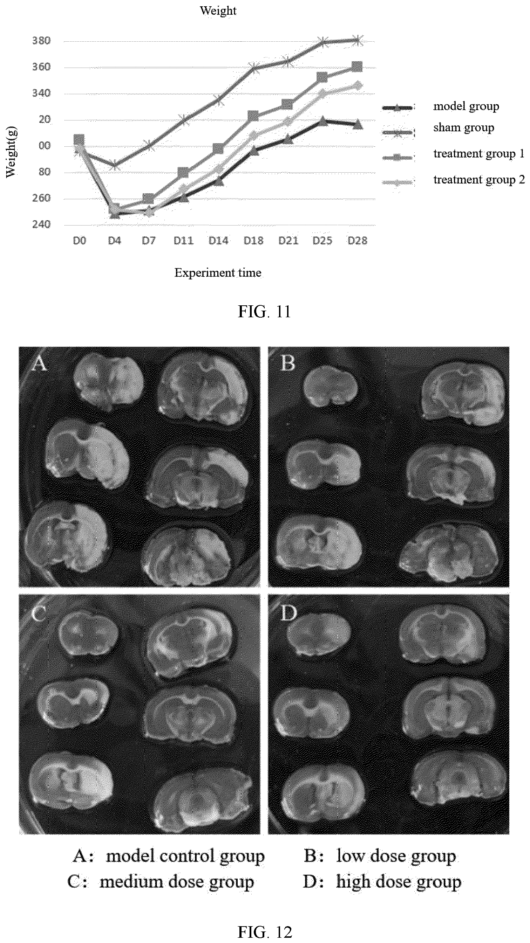

The weight results showed that the Sham-operated group (Sham group) had a normal increase in weight, with only a slight decrease in weight on the 4^{th} day after surgery. On the 4^{th} day after surgery, the weight loss of the model group and treatment group was about 1/6, indicating a significant decrease, indicating that the surgery caused significant damage to the rats. The weight recovery of the treatment group was faster than that of the model group. Statistics show that the distribution of body weight data at different time points in the model group, Sham group, and two treatment groups has homogeneity of variance. The probability value of T for the weight of the model group and Sham-operated group was 0.005 after T-test, indicating a significant difference between the two groups. This indicates that model surgery has a very significant impact on rats. As shown in the data graph, the weight recovery of the Aildenafil group (treatment group 1) is faster than that of the Sildenafil group (treatment group 2), indicating that Aildenafil is more effective in promoting the recovery of ischemic brain injury than that of the Sildenafil group. The data is shown in FIG. 11.

### ✧ Neurological deficit score

Using the nMSS neurobehavioral score, the neurological deficits and damage scores of rats after 14 days of administration are shown in the table below:

**Table 2. Scoring table for neurological deficits in rats after 14 days of administration**

| Model group | | | Sham group | | | Aildenafil group | | | Sildenafil group | | |
|---|---|---|---|---|---|---|---|---|---|---|---|
| 10 | 9 | 8 | 0 | 0 | 0 | 8 | 6 | 7 | 9 | 7 | 8 |
| 7 | 8 | 7 | 0 | 0 | 0 | 10 | 5 | 7 | 10 | 8 | 6 |
| 7 | 7 | 7 | 0 | 0 | 0 | 9 | 8 | 6 | 9 | 7 | 7 |
| 10 | 10 | 9 | 0 | 0 | 0 | 7 | 5 | 6 | 9 | 8 | 8 |
| 9 | 8 | 8 | 0 | 0 | 0 | 6 | 6 | 7 | 10 | 9 | 7 |
| 10 | 10 | 7 | 0 | 0 | 0 | 8 | 7 | 8 | 5 | 8 | 9 |
| 8 | 7 | 7 | 0 | 0 | 0 | 8 | 9 | 6 | 9 | 7 | 9 |
| 9 | 9 | 10 | 0 | 0 | 0 | 6 | 7 | 9 | 8 | 8 | 7 |
| 8 | 8 | 7 | 0 | 0 | 0 | 7 | 8 | 9 | 9 | 10 | 8 |
| 8 | 8 | 9 | 0 | 0 | 0 | 10 | 6 | 5 | 8 | 8 | 7 |

After 14 days of administration, the nerve function recovery was better in the group treated with Aildenafil group than in the group treated with Sildenafil group, with a very significant difference (p=0.01). The Aildenafil group also showed a very significant recovery compared to the model group (p=0.002). The experimental results were very surprising, indicating that Aildenafil can promote the recovery of neurological function in ischemic injury rats more effectively than Sildenafil, and there is a significant difference.

### ✧ Morris water maze experiment results

The results of the 14 days water maze test showed that the average latency period of the sham group was 4.9 seconds, the model group was 32.51 seconds, the Aildenafil group (treatment group 1) had an average latency period of 8.93 seconds, and the Sildenafil group (treatment group 2) had an average latency period of 16.88 seconds. The experiment also unexpectedly found that, compared with the model group, the latency period of the Aildenafil group was shorter, with a significant difference (P=0.03), while there was no significant difference in latency between the Sildenafil group and the model group (P=0.148), indicating that Aildenafil has a better promoting effect on the recovery of ischemic cerebral infarction than Sildenafil, and Aildenafil can significantly promote the recovery of brain memory.

### Example 22: Comparison of Therapeutic Effects of Intravenous Administration of Aildenafil Citrate and Sildenafil Citrate on Ischemic Stroke Model Rats

### 1. Test reagents and drugs

| **Reagent name** | **Source** |
|---|---|
| Isoflurane | Shenzhen Ruiwode Life Technology Co., Ltd |
| Penicillin G Sodium Salt | Phygene Company |
| Aildenafil citrate injection | Self made |
| Sildenafil citrate injection | Self made |

### 2. Testing instruments

| **Instrument name** | **Model/specificat** | **Manufacturer** |
|---|---|---|
| Electronic Balance | ECC2201 | Nanjing Bernita Scientific Instrument |
| Small animal anesthesia machine | R500IP | Ruiwode Life Technology Co., Ltd |
| Electronic balance | ME203E/02 | Mettler |

### 3. Ordering information for experimental animals

| | |
|---|---|
| Experimental animal | Rats |
| Species | SD |
| Grade | SPF grade |
| Gender | Male |
| Quantity | 50 |
| Weight | 250-270 g |
| Source | Sibeifu (Beijing) Biotechnology Co., Ltd |
| Production license number | SCXK ( 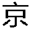 ) 2019-0010 |

### Feeding and Management

All rats were raised at a temperature of 20.5-24.5 °C, humidity of 40-75%, and a light cycle of 12 hours of light and 12 hours of darkness. A maximum of 5 rats were raised in each breeding cage, with a cage size of 48cm x 35cm x 20cm. The padding used under the cage was sterilized shaving padding, which was replaced twice a week. During the experiment, all experimental rats were allowed to eat freely, and the padding and drinking water were sterilized under high pressure and replaced twice per week. Each of the breeding cages had a corresponding abel, which is clear and detailed.

### 4. Test methods

### ✧ Model preparation

On the day of the experiment, the rat was anesthetized with isoflurane gas and placed in a supine position for fixation. The skin was cut along the midline of the neck to expose the right common carotid artery. The nerves and fascia around the blood vessels from the common carotid artery bifurcation to the base of the skull were carefully removed, and the external and internal carotid arteries were separated sequentially. The MCAO suture was inserted from the free end of the external carotid artery, and the nylon suture was introduced into the internal carotid artery from the distal end of the external carotid artery. It was inserted into the Willis ring of the middle cerebral artery to effectively block the middle cerebral artery. The length of the inserted suture was 18-20 mm away from the bifurcation of the common carotid artery. Then the free end of the external carotid artery was ligated along with the endovascular suture to prevent bleeding. The subcutaneous fascia and skin were sutured layer by layer, and penicillin was locally added dropwise to prevent infection. For the Sham group, only the internal carotid artery was isolated. 2 hours after the start of MCAO, the suture was carefully removed from the lumen of the internal carotid artery to allow for reperfusion of the internal carotid artery. Animals were kept in cages.

After 2 hours of embolization, then reperfusion, according to the scoring criteria in the attached table, the degree of neurological damage in the animals was scored using a blind method. Those with significant neurological deficits (≥ 8 points) were considered successful in modeling.

### ✧ Grouping and administration

The animals successfully modeled were randomly divided into three groups based on their scores, namely the model control group, compound 1 AD group (Aildenafil citrate group) 10mg/kg, and compound 2 XD group (Sildenafil citrate group) 10mg/kg, with 10 animals in each group. Immediate administration after 2 hours of cerebral ischemia and reperfusion, the test drug was administered intravenously once a day for 7 consecutive days. The endpoint of the experiment was 14 days after administration, and the dosage is detailed in the table below.

**Table 3**

| Group | n | Dosage | Administration method and frequency |
|---|---|---|---|
| Model control group | 10 | - | iv, qd, 7d |
| Compound 1 AD group | 10 | 10 mg/kg | iv, qd, 7d |
| Compound 2 XD group | 10 | 10 mg/kg | iv, qd, 7d |

### ✧ Mode of administration

This experiment was administered intravenously with a dosage of 10mg/kg and a volume of 5 mL/kg, once per day for consecutive 7 days.

Compound AD: dosage 10mg/kg, administration volume 5 ml/kg, i.e. formulated as 10mg/5 ml=2mg/ml; Ready to use and dispose of any remaining materials;

Compound XD: Dosage 10mg/kg, administration volume 5 ml/kg, i.e. formulated as 10mg/5 ml=2mg/ml; Prepared and used as needed, and dispose of any remaining materials.

### ✧ Detection indicators

### Neurological function score

Score before treatment, 3 days after treatment, 7 days after treatment, and 14 days after treatment.

### Determination of cerebral infarction range

At the end of the experiment, the brain tissue of the rats was collected by bloodletting and frozen in a -20°C freezer, then are sliced with a thickness of 2 mm per slice. The brain tissue slices were placed in a 2% red tetrazolium (TTC) solution and incubated at 37 °C for 5 minutes. The infarcted tissue appeared white, while the non-infarcted tissue appeared red. Image J software was used to measure the area of cerebral infarction and calculate the percentage of infarct area to the total brain area.

### ✧ Data analysis methods

The measurement data is expressed in *x̅* ± s and compared pairwise using TTEST, with a statistical difference of P<0.05.

### 5. Test results

### The impact on behavioral scores in rats

Compared with the model control group, the AD10mg/kg group showed significantly improved behavioral scores (P<0.05) after 7 days and the 14 days administration. The XD10mg/kg group showed an improvement trend, but no statistical difference was observed. The results are shown in Table 4.

**Table 4. Effects of compounds on behavioral scores in rats (x̅ ± s, n=10)**

| Group | Dosage | Before medication | 3 days after medication | 7 days after medication | 14 days after medication |
|---|---|---|---|---|---|
| Model control group | - | 9.4±0.5 | 7.9±0.9 | 6.7±1.3 | 6.0±1.4 |
| AD group | 10 mg/kg | 9.7±0.5 (*P*=0.20) | 6.9±1.5 (*P*=0.09) | 5.5±1.1* (*P*=0.04) | 4.3±1.3* (*P*=0.01) |
| XD group | 10 mg/kg | 9.6±0.5 (*P*=0.40) | 7.4±1.4 (*P*=0.36) | 6.1±1.7 (*P*=0.39) | 4.9±1.7 (*P*=0.14) |

### The impact on the range of cerebral infarction in rats

Compared with the model control group, the improvement rate of cerebral infarction range in the AD10mg/kg group was 29.6% (P<0.05) after 14 days administration. The results are shown in Table 5.

**Table 5. Effects of compounds on the range of cerebral infarction in rats (x̅ ± s, n=10)**

| Group | Dose | Total cerebral infarction% | improvement rate% |
|---|---|---|---|
| Model control group | - | 25.0±5.4 | |
| AD group | 10mg/kg | 17.6±7.7* (*P*=0.02) | 29.6 |
| XD group | 10mg/kg | 21.5±8.6 (*P*=0.29) | 14.0 |

The experimental results were unexpected: Aildenafil citrate had a statistically significant therapeutic effect on stroke model rats, significantly improving their neurological function and reducing the range of cerebral infarction. However, Sildenafil citrate had no statistically significant therapeutic effect on stroke model rats, failing to significantly improve neurological function in stroke model rats or significantly reduce the range of cerebral infarction.

### Example 23: Comparative Study on the Repair of Nerve Damage in MCAO Rats by Oral Administration of Aildenafil Citrate, Yukinafil Citrate, and Tadalafil

### 1. Experimental purpose

The reparative effects of three different compounds (Aildenafil citrate, Yukinafil citrate, Tadalafil) on nerve damage in MCAO rats were evaluated.

### 2. Experimental materials

### a) Experimental drugs

Aildenafil citrate was self-made, with a purity of>99%. Physical and chemical properties: white powder, solid, slightly soluble in water, soluble in DMF or DMSO, storage method: sealed at room temperature, storage period: 2 years.

Yukinafil citrate was self-made, with a purity of>99%. Physical and chemical properties: white powder, solid, soluble in water, soluble in DMF or DMSO, storage method: sealed at room temperature, storage period: 2 years.

Tadalafil was self-made, with a purity of>99%. Physical and chemical properties: white powder, solid, insoluble in water, soluble in DMF or DMSO, storage method: sealed at room temperature, storage period: 2 years.

### b) Experimental animals

SD rats (purchased from Sibeifu (Beijing) Biotechnology, 210-230g, male). After purchasing, the rats were adaptively raised in the laboratory for 1-2 weeks, followed by subsequent experiments.

### 3. Experimental methods and processes

### a) Drug preparation

Preparation of Aildenafil Citrate: accurately weighing 1600mg of Aildenafil Citrate drug, dissolving in 800 ml of pure water to prepare a 2mg/ml drug solution, refrigerating at 4 °C for later use.

Preparation of Yukinafil Citrate: accurately weighing 1600mg of Yukinafil Citrate drug, dissolving in 800 ml of pure water to prepare 2mg/ml drug suspension solution, and refrigerating at 4°C for later use.

Preparation of Tadalafil: accurately weighing 1600mg of Tadalafil drug, and dissolving in 800 ml of 5% CMC Na to prepare a 2mg/ml drug suspension solution, which is prepared on site as needed.

### b) Administration route, Administration dosage, and Administration time

Administration route: gavage administration;
Dosage: 8mg/kg body weight, twice a day;
Administration time: gavage administration twice a day in the morning and evening (administered immediately after modeling).

### c) Production of MCAO models

A rat model of middle cerebral artery ischemia/reperfusion (MCAO/R) was created using suture-occluded method.

After 2 hours of embolization, then reperfusion, according to the scoring criteria in the attached table, the degree of neurological damage in the animals was scored using a blind method. Those with significant neurological deficits (≥ 8 points) were considered successful in modeling.

### d) Experimental grouping

The successfully modeled rats were randomly divided into 4 groups based on their scores, with an average of 20 rats in each group. They were labeled as MCAO model group (referred to as model group), Aildenafil treatment group 1, Yukinafil treatment group 2, and Tadalafil treatment group 3, respectively.
1) MCAO model group: cerebral ischemia-reperfusion group;
2) Treatment group 1: MCAO model+ Aildenafil citrate drug treatment group;
3) Treatment group 2: MCAO model+Yukinafil citrate drug treatment group;
4) Treatment group 3: MCAO model+Tadalafil drug treatment group;

### e) Experimental measurement indicators

Neurological function score: evaluation was conducted before treatment, 3 days, 7 days, and 14 days after treatment.

### Determination of cerebral infarction range

At the end of the experiment, the brain tissue of the rats was collected by bloodletting and frozen in a -20 °C freezer, and then is sliced into a thickness of 2 mm per slice. The brain tissue slices were placed in a 2% red tetrazolium (TTC) solution and incubated at 37°C for 5 minutes. The infarcted tissue appeared white, while the non-infarcted tissue appeared red. Image J software was used to measure the area of cerebral infarction and the percentage of infarct area to the total brain area was calculated.

### f) Data analysis methods

The measurement data was expressed in *x̅* ± s and compared pairwise using TTEST, with a statistical difference of P<0.05.

### 4. Test results

### ✧ The impact on behavioral scores of rats

Compared with the model control group, the Aildenafil group showed significant improvement in behavioral scores after 3, 7, and 14 days administration (P<0.05). There was a trend of improvement between the Yukinafe group and the Tadalafil group, but no statistically significant difference was observed. after 14 days administration, the group treated with Aildenafil was more effective than the groups treated with Yukinafil and Tadalafil, with a significant difference (P=0.037 between the Aildenafil and Sildenafil groups and P=0.026 between the Aildenafil and Tadalafil groups).

### ✧ The impact on the range of cerebral infarction in rats

Compared with the model control group, the improvement rate of cerebral infarction range in the Aildenafil group after 14 days was 31.5% (P<0.05). The results are shown in Table 6.

**Table 6. Effects of compounds on the range of cerebral infarction in rats (x̅ ± s, n=20)**

| Group | Dosage | Total cerebral infarction% | Improvement rate% |
|---|---|---|---|
| Model control group | - | 24.8±5.3 | |
| Aildenafil group | 8mg/kg*2 | 17.0±6.7* | 31.5 |
| Yukinafil group | 8mg/kg*2 | 21.2±6.3 | 13.1 |
| Tadalafil group | 8mg/kg*2 | 21.3±6.5 | 14.2 |

### Note:

1. Compared with the model control group, the P value of the Aildenafil group was 0.0002, * P<0.05, indicating that the Aildenafil group could significantly improve the range of cerebral infarction;
2. Compared with the model group, the Yukinafil group and Tadalafil group had a P>0.05, indicating a trend of improving the range of cerebral infarction between the Yukinafil group and Tadalafil group, but no significant statistical difference was observed;
3. Compared with the group treated with Yukinafil, the P value of the group treated with Aildenafil was 0.035, with P<0.05, indicating that Aildenafil is also significantly more effective than Yukinafil;
4. Compared with the Tadalafil group, the P value of the Aildenafil group is 0.048, and P<0.05, indicating that Aildenafil is also significantly more effective than Tadalafil.

### Example 24: Study on the Efficacy of Injection of Different Doses of Aildenafil Phosphate in MCAO Rats

### 1. Experimental design

✧ Animal selection: healthy SD rats were used as experimental animals. In order to eliminate the impact of individual animal variations on the results, 10 rats were used in each group. The experimental process strictly follows the requirements of the protocol to ensure animal welfare.
✧ Dosage and administration route: The low-dose 2.5 mg/kg group, medium dose 5 mg/kg group, and high-dose 10 mg/kg group of the test drug were administered with Aildenafil phosphate (code ADLS1026). After 2 hours of cerebral ischemia-reperfusion, the drug was immediately injected into the tail vein for consecutive 7 days.

### 2. Test materials

✧ Test substance: Aildenafil phosphate; Batch number: 20220908.
✧ Test reagents:

| Reagent name | Batch number | **Source** |
|---|---|---|
| Penicillin G sodium | C12854341 | McLean |
| Red tetrazolium (TTC) | 20210220 | Phygene |
| Isoflurane | 20220202 | Rayward |

✧ Testing instruments

| Instrument name | Model/specification | Manufacturer |
|---|---|---|
| Electronic Balance | ECC2201 | Nanjing Bernita Scientific Instrument Co., Ltd |
| Electronic Balance | ME203E | Mettler |
| Small animal anesthesia | R500IP | Ruiwode Life Technology Co., Ltd |

✧ Experimental animals

| Experimental animals | Healthy rats |
|---|---|
| Species | SD |
| Grade | SPF |
| Gender | Male |
| Quantity | 60 |
| Weight | 240-260g |
| Source | Sibeifu (Beijing) Biotechnology Co., Ltd |
| Production license number | SCXK ( 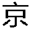 ) 2019-0010 |
| Quality certificate number | 110324220106384338 |

All rats were raised at a temperature of 20.5-24.5 °C, humidity of 40-75%, and a light cycle of 12 hours of light and 12 hours of darkness. A maximum of 5 rats can be raised in each breeding cage, with a cage size of 48 cm x 35 cm x 20 cm. The padding used under the cage was sterilized shaving padding, which was replaced twice per week. During the experiment, all experimental rats were allowed to eat freely, and the padding and drinking water were sterilized under high pressure and replaced twice per week.

### 3. Test methods

### ✧ Model preparation

On the day of the experiment, the rat was anesthetized with isoflurane gas and fixed in a supine position. The skin was cut along the midline of the neck to expose the right arteriae carotis communis. The nerves and fascia around the blood vessels from the common carotid artery bifurcation to the base of the skull were carefully removed, and the external and internal carotid arteries were separated sequentially. A MCAO suture (250-280g) was inserted from the free end of the external carotid artery, and the nylon suture was introduced into the internal carotid artery from the distal end of the external carotid artery. It was inserted into the middle cerebral artery in the Willis ring to effectively block the middle cerebral artery. The length of the inserted suture was 18-20mm away from the bifurcation of the common carotid artery. Then the free end of the external carotid artery was ligated along with intracavity suture to prevent bleeding. The subcutaneous fascia and skin were sutured layer by layer, and penicillin was locally added dropwise to prevent infection. The Sham group only isolated the internal carotid artery. 2 hours after the start of MCAO, the suture was carefully removed from the lumen of the internal carotid artery to allow for reperfusion of the internal carotid artery. Animals were kept in cages.

### ✧ Grouping and administration

The rats successfully modeled with a neurological function score of 8-12 were randomly divided into four groups based on the score: model control group, low-dose 2.5 mg/kg group of test drug, medium dose 5 mg/kg group of test drug, and high-dose 10 mg/kg group of test drug, with 7 rats in each group. The test drug group was administrated Aildenafil phosphate (code ADLS1026), while the model control group was administrated an equal volume of solvent. Immediately after cerebral ischemia-reperfusion for 2 hours, the drug was administered via tail vein injection for consecutive 7 days. The endpoint of the experiment is 14 days after the first administration. Please see Table 7 for details.

**Table 7 Grouping Information Table**

| Group | n | Dosage (mg/kg) | Administration method/volume | Administration frequency |
|---|---|---|---|---|
| Model control group | 7 | - | iv/2ml/kg | qd, 7d |
| Low dose group of test drug | 7 | 2.5 | iv/2ml/kg | qd, 7d |
| Mid dose group of test drug | 7 | 5 | iv/2ml/kg | qd, 7d |
| High dose group of test drug | 7 | 10 | iv/2ml/kg | qd, 7d |

### ✧ Detection indicators

(1) Balance beam and forelimb placement detection

Balance beam and forelimb placement tests were performed after 7 and 14 days administration, respectively.

### (2) Cerebral infarction range (TTC)

After 14 days of medication, rats were anesthetized with isoflurane gas to collect blood and euthanized by cervical vertebrae dislocation. The brain tissue was taken and placed in a -20°C freezer for freezing, and was sliced from front to back, each slice has a thickness of 2 mm. Brain tissue slices were incubated in a 2% red tetrazolium (TTC) solution at 37 °C for 5 minutes. The infarcted tissue appears white, while the non-infarcted tissue appears red. Image J software was used to measure the area of cerebral infarction and the percentage of infarct area to the total brain area was calculated.

### (3) Data analysis methods

Using the DPS data processing system of Zhejiang University for analysis, the quantitative data was represented by *x̅*± s, LSD test was used for comparison of homogeneous variance data, and non-parametric Kruskal Wallis test was used for non-homogeneous variance data. P<0.05 was statistically significant.

### 4. Test results

### ✧ Balance beam and forelimb placement test

In the balance beam test of rats, the ischemic cerebral stroke model group showed significant neurological symptoms in the paralyzed limbs, such as gait sideslip, falling during walking, and so on. Compared with the model control group, the ADLS1026 medium and high dose groups (5 mg/kg, 10 mg/kg) significantly improved the walking ability of rats on the balance beam 14 days after treatment (P<0.05), as shown in Table 8.

In the forelimb placement test of rats, the ischemic cerebral stroke model group showed significant obstacles in placing the paralyzed limbs, such as no lifting or fewer lifting times. Compared with the model control group, the ADLS1026 medium and high dose groups (5 mg/kg, 10 mg/kg) improved the forelimb placement rate of rats 14 days after treatment (P<0.05). The results are shown in Table 9.

**Table 8: Effect of ADLS1026 on the treatment balance beam of acute ischemic stroke in rats (x̅ ± sD, n=7)**

| Group | Dosage (mg/kg) | 7 days after medication (min) | 14 days after medication (min) |
|---|---|---|---|
| Model control group | - | 1.57±0.98 | 2.14±1.68 |
| Low dose group | 2.5 | 1.57±0.79 | 2.86±1.35 |
| Medium dose group | 5 | 2.14±0.9 | 4.29±1.38* |
| High dose group | 10 | 2.71±1.25 | 4.29±1.60* |

| | | | |
|---|---|---|---|
| Note: Compared with the model control group: * P < 0.05. | | | |

**Table 9 Effects of ADLS1026 on Forelimb Placement during Acute Treatment of Ischemic Stroke in Rats (x̅ ± sD, n=7)**

| Group | Dosage | 7 days after medication | | 14 days after medication | |
|---|---|---|---|---|---|
| | (mg/kg) | Forelimb left (%) | Forelimb right (%) | Forelimb left (%) | Forelimb right (%) |
| Model control group | - | 4.29±11.34 | 100±0 | 8.57±12.15 | 100±0 |
| Low dose group | 2.5 | 10.00±17.32 | 100±0 | 17.14±22.15 | 100±0 |
| Medium dose group | 5 | 18.57±18.64 | 100±0 | 37.14±30.39* | 100±0 |
| Medium dose group | 10 | 14.29±18.13 | 100±0 | 38.57±28.54* | 100±0 |

| | | | | | |
|---|---|---|---|---|---|
| Note: Compared with the model control group: * P < 0.05. | | | | | |

### ✧ The impact on the range of cerebral infarction in rats

Ischemic cerebral stroke model rats showed significant cerebral infarction. Compared with the model control group, different dose groups of ADLS1026 can improve the range of cerebral infarction in model rats in varying degrees. After 14 days administration, the improvement rates of the low (2.5 mg/kg), medium (5 mg/kg), and high (10 mg/kg) dose groups were 22.6% (P>0.05), 46.5% (P<0.05), and 43.4% (P<0.05), respectively. The results are shown in Table 10 and FIG. 12.

**Table 10: Effect of ADLS1026 on the range of cerebral infarction treated with acute ischemic stroke in rats (x̅ ± sD, n=7)**

| Group | Dosage (mg/kg) | Infarct size (%) | Improvement rate (%) |
|---|---|---|---|
| Model control group | - | 24.54±9.01 | - |
| Low dose group | 2.5 | 19.00±7.76 | 22.6 |
| Medium dose group | 5 | 13.13±4.12* | 46.5 |
| High dose group | 10 | 13.90±4.70* | 43.4 |

| | | | |
|---|---|---|---|
| Note: Compared with the model control group: * P < 0.05. | | | |

## Claims

1. Use of a compound with formula (I), pharmaceutically acceptable salts thereof, or pharmaceutical compositions comprising them in the preparation of a drug for treatment or prevention of human diseases caused by cerebral ischemia:

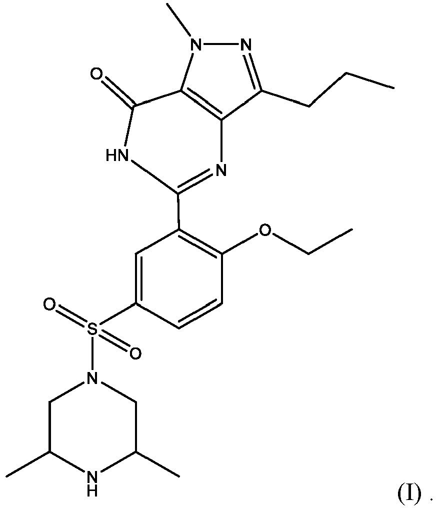

2. The use according to claim 1, **characterized in that**, the use of Aildenafil or its pharmaceutically acceptable salts or drug compositions containing them in the preparation of drugs for the treatment or prevention of human local ischemic cerebral infarction, human ischemic cerebral stroke, human local cerebral ischemic memory impairment, and senile dementia, human energy metabolism depletion of global cerebral ischemia, or human cerebral ischemic neurological symptoms caused by brain trauma.

3. The use according to claim 1, **characterized in that**, the pharmaceutically acceptable salts of Aildenafil are citrate, sulfate, hydrochloride, phosphate, tartrate or semi-tartrate, nicotinate, lactate, gluconate, maleate or aspartate; preferably citrate, sulfate, hydrochloride, phosphate, tartrate or semi-tartrate.

4. The use according to claim 3, **characterized in that**, the tartrate or semi-tartrate of Aildenafil is D-tartrate or semi-D-tartrate, L-tartrate or semi L-tartrate, DL-tartrate or semi DL-tartrate, or meso tartrate or semi meso tartrate; preferably, Aildenafil D-tartrate or semi-D-tartrate, or Aildenafil L-tartrate or semi-L-tartrate.

5. The use according to claim 1, **characterized in that**, the pharmaceutical composition comprising Aildenafil or pharmaceutically acceptable salts thereof is an oral preparation or injectable preparation; preferably tablets, oral solutions, or injection solutions.

6. The use according to claim 5, **characterized in that**, the effective dosage used for human of the compound is 0.1~20mg/kg/day, administrated once or multiple times per day; preferably, the oral dosage used for human is 0.2mg/kg/day~10mg/kg/day, and the injection dosage used for human is 0.1mg/kg/day~5mg/kg/day.

7. A pharmaceutically acceptable salt of Aildenafil according to claim 1, **characterized in that**, the pharmaceutically acceptable salts of Aildenafil are Aildenafil phosphate or Aildenafil tartrate.

8. The pharmaceutically acceptable salt of Aildenafil according to claim 7, **characterized in that**, Aildenafil tartrate is D-tartrate, L-tartrate, DL-tartrate, or meso tartrate; preferably Aildenafil D-tartrate or Aildenafil L-tartrate.

9. A method for preparing the compounds according to any one of claims 1-8, **characterized in that**, the method comprises cyclization of 4-[2-ethoxy-5-(cis-3,5- dimethylpiperazine-1-sulfonyl) benzamide]-1-methyl-3-n-propylpyrazole-5-formamide to synthesize Aildenafil, wherein the base catalyst is sodium tert-butoxide.
